# EUROPÄISCHE PATENTANMELDUNG

(11) **EP 4 464 281 A1**
(43) Veröffentlichungstag der Anmeldung: **20.11.2024**
(21) Anmeldenummer: 24172182.8
(22) Anmeldetag: 24.04.2024
(51) Int. Cl.: A61F 2/46, A61B 17/88, A61F 2/30

(54) **MEDIZINISCHER APPLIKATOR UND VERFAHREN ZUM BELADEN EINES MEDIZINISCHEN APPLIKATORS**

(30) Priorität: 17.05.2023 DE 102023113068
(71) Anmelder: Karl Storz SE & Co. KG, 78532 Tuttlingen (DE)
(72) Erfinder: Pfeffer, Marian, 78532 Tuttlingen (DE); Sterk, Seraina, 78532 Tuttlingen (DE)

(57) **Zusammenfassung**

Die Erfindung betrifft einen medizinischen Applikator (10) zum Applizieren eines Mittels, umfassend einen Grundkörper (12), einen an dem Grundkörper (12) angeordneten Schaft (14), wobei der Grundkörper (12) und der Schaft (14) einen Kanal (16) ausbilden, der sich durch den Grundkörper (12) und den Schaft (14) hindurch erstreckt und durch den das Mittel einer Applikationsstelle zuführbar ist, einen Stößel (18), der in den Kanal (16) einschiebbar ist, um das Mittel zu applizieren, ein Betätigungselement (20), das an dem Grundkörper (12) geführt bewegbar ist, und einen Aufnahmeraum (22), der von dem Grundkörper (12) und/oder dem Betätigungselement (20) ausgebildet ist und der dazu eingerichtet ist, das Mittel aufzunehmen, wobei das Betätigungselement (20) durch eine Relativbewegung zu dem Grundkörper (12) in eine Applikationsposition bringbar ist, wobei der Aufnahmeraum (22) durch die Relativbewegung zwischen dem Betätigungselement (20) und dem Grundkörper (12) verkleinerbar ist, und wobei in den Aufnahmeraum (22) eingebrachtes Mittel durch das Verkleinern des Aufnahmeraums (22) dem Kanal (16) zuführbar ist.

## Beschreibung

Die vorliegende Erfindung betrifft einen medizinischen Applikator zum Applizieren eines Mittels und ein Verfahren zum Beladen eines medizinischen Applikators.

Knorpelgewebe ist ein wichtiges Gewebe im menschlichen Körper, das eine tragende Rolle bei der Bewegung und Stabilität von Gelenken spielt. Es ist jedoch anfällig für Verschleiß und Verletzungen. Infolge von Verletzungen oder Erkrankungen wie Arthritis kann Knorpelgewebe abgebaut werden, was zu Gelenkschäden und in weiterer Folge zu Schmerzen, Einschränkungen der Beweglichkeit und anderen negativen Folgen führen kann.

Bisherige Verfahren zur Herstellung von Knorpelgewebeersatzstoffen und Behandlung von Gelenkschäden sind aufwendig und zeitintensiv, was zu hohen Kosten und begrenzten Verfügbarkeiten führt. Es besteht daher Bedarf an einem schnellen und effizienten Verfahren, um den Bedarf an Regenerationshandlungen abzudecken.

Ein vielversprechendes Verfahren für die Behandlung von Gelenkschäden stellt das "Minced Cartilage"-Verfahren dar. Das "Minced Cartilage"-Verfahren ist eine innovative Technik zur Behandlung von Gelenkschäden, die in den letzten Jahren zunehmend an Popularität gewonnen hat. Insbesondere wird das Verfahren häufig bei Patienten mit Knorpelschäden im Kniegelenk eingesetzt, kann aber auch zur Behandlung von Knorpelschäden anderer Gelenke wie dem Sprunggelenk oder dem Ellenbogen verwendet werden.

Das Verfahren basiert auf der Verwendung von körpereigenem Knorpelgewebe, das aus dem betroffenen Gelenk entnommen wird. Dieser Knorpel wird in kleine Stücke zerkleinert und die Knorpelstücke dann zusammen mit Blutplättchen und Wachstumsfaktoren des Patienten, die aus einer Blutprobe gewonnen werden, zu einem Knorpel-Konzentrat verarbeitet. Das Konzentrat wird schließlich mittels eines medizinischen Applikators auf den defekten Bereich des Knorpels aufgebracht, um den defekten Knorpel zu reparieren.

Eine der Hauptvorteile dieser Methode ist, dass sie minimalinvasiv ist und normalerweise ambulant durchgeführt werden kann. Im Vergleich zu traditionellen operativen Verfahren zur Behandlung von Knorpelschäden ist das "Minced Cartilage"-Verfahren für den Patienten schonender und erfordert eine kürzere Erholungszeit. Patienten können normalerweise innerhalb weniger Wochen nach dem Eingriff wieder mobil sein.

Eine weitere wichtige Eigenschaft des "Minced Cartilage"-Verfahrens ist, dass sie auf körpereigenen Materialien basiert. Da das verwendete Knorpelgewebe aus dem eigenen Körper des Patienten stammt, besteht keine Gefahr von Abstoßungsreaktionen oder Infektionen. Im Gegensatz zu anderen Verfahren wie der Verwendung von synthetischen Implantaten oder Transplantaten aus Spendergewebe gibt es bei dieser Methode keine Risiken im Zusammenhang mit der Kompatibilität.

Zur Durchführung des "Minced Cartilage" Verfahrens gibt es spezielle medizinische Applikatoren. Diese bestehen in der Regel aus einem langen, schmalen Schaft mit einem speziell geformten Ende, das je nach Größe und Form des betroffenen Gelenks angepasst werden kann. Das Ende des Schafts hat eine Öffnung und einen kleinen Durchmesser. Es wird verwendet, um das Konzentrat auf den defekten Bereich des Gelenks aufzubringen. Um das Konzentrat durch den Schaft zu transportieren, umfassen die Applikatoren häufig einen Stößel, der durch den Schaft bewegbar ist. Nachdem ein Applikator über einen Zugangspunkt, der durch eine minimalinvasive Arthroskopie geschaffen wurde, in das Gelenk eingeführt wurde, kann das Konzentrat appliziert werden. Dazu wird durch sanften Druck auf den Stößel des Applikators das Konzentrat aus dem Ende des Schafts herausgedrückt und auf den beschädigten Bereich aufgebracht.

Um das Konzentrat in den Applikator laden zu können, gibt es verschiedene Methoden. Beispielsweise gibt es Applikatoren, deren proximales Ende des Schafts offen ist und zum Beladen des Konzentrats eingerichtet ist. Andere Applikatoren weisen etwa ein Beladefenster auf, das sich zu einer medialen Seite des Applikators öffnet und durch das das Konzentrat in den Schaft geladen werden kann. Nach dem Beladen muss das Beladefenster mittels eines Deckels verschlossen werden, um das Konzentrat mittels des Stößels applizieren zu können. In beiden Fällen ist das Beladen jedoch nicht bequem, schnell, effizient und sauber durchzuführen. Beispielsweise ist es nachteilig, nach dem Beladen den Deckel auf das Beladefenster aufbringen zu müssen. Dadurch kann es zu einer Kontamination des Konzentrats kommen und/oder eine Außenfläche des Applikators durch herausquellendes Konzentrat verschmutzt werden. Auch das Befüllen über das proximale Ende des Schafts kann zu einer Kontamination des Konzentrats, Volumenverlust des Konzentrats und/oder zu einer Verschmutzung des Applikators führen. Zudem ist hierbei das Einführen mühsam, weil das Konzentrat in einen engen Kanal eingebracht werden muss.

Ausgehend vom Stand der Technik liegt der Erfindung die Aufgabe zugrunde, einen medizinischen Applikator mit großer Benutzerfreundlichkeit und hoher Effizienz bereitzustellen.

Diese Aufgabe wird erfindungsgemäß gelöst durch einen medizinischen Applikator zum Applizieren eines Mittels und ein Verfahren zum Beladen eines medizinischen Applikators, wie sie hierin beschrieben und in den Ansprüchen definiert sind.

Die vorliegende Erfindung sieht vor, einen medizinischen Applikator zum Applizieren eines Mittels bereitzustellen. Der Applikator umfasst einen Grundkörper, einen an dem Grundkörper angeordneten Schaft, wobei der Grundkörper und der Schaft einen Kanal ausbilden, der sich durch den Grundkörper und den Schaft hindurch erstreckt und durch den das Mittel einer Applikationsstelle zuführbar ist. Zudem umfasst der Applikator einen Stößel, der in den Kanal einschiebbar ist, um das Mittel zu applizieren, ein Betätigungselement, das an dem Grundkörper geführt bewegbar ist, und einen Aufnahmeraum, der von dem Grundkörper und/oder dem Betätigungselement ausgebildet ist und der dazu eingerichtet ist, das Mittel aufzunehmen, wobei das Betätigungselement durch eine Relativbewegung zu dem Grundkörper in eine Applikationsposition bringbar ist, wobei der Aufnahmeraum durch die Relativbewegung zwischen dem Betätigungselement und dem Grundkörper verkleinerbar ist, und wobei in den Aufnahmeraum eingebrachtes Mittel durch das Verkleinern des Aufnahmeraums dem Kanal zuführbar ist.

Vorteilhafterweise kann derart ein Applikator mit großer Benutzerfreundlichkeit und hoher Effizienz bereitgestellt werden. In einfacher und effizienter Weise kann der Applikator mit dem Mittel beladen werden. Das Beladen wird schneller und sauberer. Dadurch kann zudem eine Kontamination des Mittels vermieden werden. Weiterhin kann ein Materialverlust reduziert werden, was beispielsweise die Kosten einer Durchführung einer Behandlung mittels eines Applikators kostengünstiger macht. Vorteilhafterweise kann das Mittel in den Aufnahmeraum eingebracht werden und durch das Verkleinern dem Kanal zugeführt werden. Dadurch ist der Applikator besonders benutzerfreundlich. Ein Benutzer hat einen relativ großen Aufnahmeraum zum Einbringen des Mittels zur Verfügung und kann diesen verkleinern, wenn er das Mittel applizieren möchte. Ferner ist ein derartiger Applikator kostengünstig herzustellen und kommt im Wesentlichen ohne kleine bzw. diffizil handzuhabende Teile wie etwa einen Deckel aus. Die Benutzung des erfindungsgemäßen Applikators ist sauberer und effizienter als bei herkömmlichen Applikatoren. Zum einen gibt es einen relativ großen Aufnahmeraum, der ein Einbringen des Mittels vereinfacht, und zum anderen kann das Mittel einfach und effizient dem Kanal zugeführt werden, indem der Aufnahmeraum verkleinert wird. Allgemein kann dadurch auch ein benötigter Bauraum reduziert werden und ein kostengünstig zu fertigender Applikator bereitgestellt werden.

Der Applikator kann insbesondere ein Applikator zum Applizieren eines Mittels zur Durchführung des "Minced Cartilage" Verfahrens sein. Ferner kann der Applikator auch zur Durchführung anderer Verfahren geeignet sein. Es kann vorgesehen sein, dass der Applikator zur Durchführung anderer Verfahren umrüstbar und/oder eingerichtet ist. Beispielsweise kann der Schaft zur Durchführung eines bestimmten Verfahrens ausgebildet sein. Dieser Schaft kann zur Verwendung mit dem Applikator vorsehbar sein. Generell ist der Applikator dazu geeignet, das Mittel zu applizieren. Applizieren kann in dem Zusammenhang aufbringen, einbringen, auftragen, injizieren und/oder dergleichen bedeuten. Beispielsweise kann das Mittel auf einen defekten Bereich eines Gelenks aufgebracht werden. Dieser Bereich kann die Applikationsstelle sein. Das kann bedeuten, dass das Mittel in einen Gelenkraum injiziert und/oder eingebracht wird. Grundsätzlich wird bei dem Applizieren das Mittel aus einem distalen Ende des Schafts hinaus, insbesondere zu der Applikationsstelle, befördert.

Das Mittel kann insbesondere ein Stoffgemisch sein. Das Stoffgemisch kann insbesondere ein Stoffgemisch sein, das zur Durchführung des "Minced Cartilage" Verfahrens bereitgestellt wird. Das kann beispielsweise bedeuten, dass das Mittel Gewebestücke, insbesondere Knorpelgewebestücke, vorzugsweise des Patienten umfasst, bei dem das Mittel appliziert werden soll. Weiterhin kann das Mittel Bestandteile des Blutes des Patienten umfassen. Das Mittel kann zudem ein homogenes oder heterogenes Mittel sein. Weiterhin kann das Mittel mehrere Phasen umfassen. Isbesondere kann das Mittel Festkörper und/oder festkörperartige Phasen und/oder Stücke umfassen. Zudem kann das Mittel zumindest eine flüssige und/oder wässrige Phase umfassen. Weiterhin kann das Mittel komprimierbar sein. Das kann bedeuten, dass ein Volumen des Mittels reduzierbar ist. Das Mittel kann hierfür zumindest einen kompressiblen Bestandteil umfassen. Beispielsweise können die Gewebestücke, insbesondere die Knorpelgewebestücke, komprimierbar sein. Das kann bedeuten, dass die Gewebestücke, insbesondere die Knorpelgewebestücke, gepresst und/oder gedrückt werden können.

Der Grundkörper kann einen Teil, insbesondere einen Großteil, der Außenfläche des Applikators definieren. Beispielsweise kann der Grundkörper 20 % - 100 %, insbesondere 40 % - 90 %, bevorzugt 60 % - 80 %, der Außenfläche des Applikators definieren. Ferner kann der Grundkörper dazu vorgesehen sein, von einem Benutzer gehalten zu werden. Das kann bedeuten, dass der Grundkörper dazu vorgesehen ist, von dem Benutzer während des Applizierens gehalten zu werden. Dazu kann der Grundkörper eine ergonomische Formgebung aufweisen. Das kann beispielsweise bedeuten, dass der Grundkörper Außenmaße aufweist, die derart beschaffen sind, dass der Benutzer den Grundkörper, insbesondere den Applikator, mit einer Hand halten kann. Allgemein kann der Grundkörper einen Hauptkörper des Applikators definieren. Ein für ein menschliches Auge wahrnehmbares Erscheinungsbild des Applikators kann maßgeblich durch den Grundkörper definiert sein. Zudem kann der Grundkörper einfach reinigbar, desinfizierbar und/oder autoklavierbar sein. Es kann auch vorgesehen sein, dass der Grundkörper und/oder der gesamte Applikator für einen einmaligen Gebrauch vorgesehen ist/sind. Außerdem kann sich der Grundkörper zumindest im Wesentlichen entlang einer Haupterstreckungsrichtung erstrecken. Unter einer "Haupterstreckungsrichtung" eines Objekts soll insbesondere eine Richtung verstanden werden, welche parallel zu einer längsten Kante eines kleinsten gedachten Quaders verläuft, welcher das Objekt gerade noch vollständig umschließt und welche vorzugsweise durch einen geometrischen Mittelpunkt und/oder durch einen Massenmittelpunkt des Bauteils verläuft.

Der Schaft kann länglich ausgebildet sein und/oder sich zumindest im Wesentlichen entlang der Haupterstreckungsrichtung des Grundkörpers erstrecken. Der Schaft kann sich zumindest teilweise von einem distalen Ende des Grundkörpers in die distale Richtung entlang der Haupterstreckungsrichtung des Grundkörpers erstrecken. Zudem kann der Schaft stabförmig und/oder rohrförmig ausgebildet sein. Das kann bedeuten, dass ein Außendurchmesser des Schafts im Verhältnis zu der Länge des Schafts klein ist. Bevorzugt ist der Schaft dazu eingerichtet, in einen Körper des Patienten, insbesondere zum Applizieren des Mittels, eingeführt zu werden. Der Schaft kann hohlzylinderförmig ausgebildet sein und/oder an seinem distalen Ende eine Öffnung umfassen, durch die das Mittel aus dem Schaft beförderbar ist. Insbesondere ist die Öffnung für das Applizieren derart in einen Nahbereich zu der Applikationsstelle bringbar, dass das Mittel der Applikationsstelle zuführbar ist. In dem Zusammenhang kann mit Zuführen Applizieren gemeint sein.

Die Applikationsstelle kann sich insbesondere innerhalb eines Körpers, insbesondere innerhalb eines Knies, des Patienten befinden. Die Applikationsstelle kann insbesondere ein Defekt, insbesondere eines Knorpelgewebes, sein. Der Schaft kann teilweise innerhalb des Grundkörpers angeordnet sein. Insbesondere kann der Grundkörper eine Schaftmontageausnehmung aufweisen, in die der Schaft montierbar und/oder montiert ist. Der Schaft kann insbesondere lösbar an dem Grundkörper angeordnet sein. In einigen Ausführungsformen ist der Schaft an den Grundkörper angeklebt. Der Schaft kann auswechselbar sein und/oder für eine Patientenanatomie und/oder eine bestimme Handlung bestimmt ausgebildet sein. Beispielsweise kann der Schaft eine Biegung in einem distalen Endbereich des Schafts aufweisen. Die Biegung kann derart ausgebildet sein, dass das Mittel in einem Winkel von beispielsweise 0° bis 120°, insbesondere 5° bis 90°, bevorzugt 10° bis 70° und besonders bevorzugt 10° bis 45° aus dem Schaft ausbringbar ist. Ferner kann der Schaft anwendungs- und/oder patientenspezifisch eine unterschiedliche Länge aufweisen. Generell kann sich der Schaft über beispielsweise 5 cm bis 40 cm, insbesondere 10 cm bis 30 cm, vorzugsweise 15 cm bis 25 cm entlang der Haupterstreckungsrichtung erstrecken, beziehungsweise derart lang sein. Ein Außendurchmesser des Schafts kann beispielsweise 0.5 cm bis 5 cm, insbesondere 0.5 cm bis 3 cm, bevorzugt 1 cm bis 2 cm betragen.

Unter dem Kanal kann ein gesamter gedachter, insbesondere zylindrischer und vorzugsweise kreiszylindrischer, Raum verstanden werden, durch den der Stößel schiebbar ist. Der Kanal kann sich durch den Aufnahmeraum hindurch erstrecken. Der Kanal muss insbesondere nicht zwangsläufig entlang seiner gesamten Länge durch Wandungen und/oder dergleichen begrenzt sein. Der Kanal kann beispielsweise in den Aufnahmeraum übergehen und/oder sich ohne physische Begrenzung durch diesen hindurch erstrecken. Insbesondere kann sich der Kanal durch den gesamten Schaft hindurch bis zu der Öffnung des Schafts erstrecken. Ein Durchmesser des Kanals entspricht insbesondere einem Innendurchmesser des Schafts. Das Mittel kann durch den Kanal förderbar sein. Der Kanal kann sich von dem proximalen Ende des Applikators zu dem distalen Ende, insbesondere der Öffnung, erstrecken. In diesem Zusammenhang kann der Kanal länger sein als der Schaft. Der Kanal kann insbesondere eine Länge von beispielsweise 5 cm bis 60 cm, insbesondere 15 cm bis 40 cm, vorzugsweise 20 cm bis 30 cm aufweisen. Ferner kann der Kanal einen Innendurchmesser von beispielsweise 0.1 cm bis 4 cm, insbesondere 0.1 cm bis 3 cm, bevorzugt 1 cm bis 2 cm aufweisen. Grundsätzlich kann der Innendurchmesser des Kanals kleiner sein als der Außendurchmesser des Schafts.

insbesondere kann zu dem Befördern des Mittels der Stößel vorgesehen sein. Der Stößel kann einen Außendurchmesser aufweisen, der zumindest im Wesentlichen einem Innendurchmesser des Schafts und/oder einem Durchmesser des Kanals entspricht. Vorzugsweise ist der Stößel zumindest im Wesentlichen passgenau zu dem Schaft ausgebildet. Das kann bedeuten, dass der Stößel einen zumindest im Wesentlichen geringfügig geringeren Außendurchmesser aufweist im Vergleich zu dem Innendurchmesser des Schafts. Durch den geringfügig geringeren Außendurchmesser kann ein Druckausgleich während des Beförderns des Mittels entlang des Kanals erreicht werden. Ferner kann hierdurch eine komfortable Bewegbarkeit des Stößels erzielt werden, weil etwa eine Reibung zwischen dem Stößel und dem Schaft klein ist. Der Stößel kann an einem proximalen Ende des Applikators dem Kanal zuführbar sein und zumindest im Wesentlichen bis zu einem distalen Endbereich des Schafts schiebbar sein. Während des Schiebens kann der Stößel den Aufnahmeraum passieren und/oder durch diesen hindurchschiebbar sein. In dem Aufnahmeraum kann sich das Mittel befinden. Indem der Stößel durch den Aufnahmeraum hindurchschiebbar ist, kann durch das Schieben des Stößels das Mittel aus dem Aufnahmeraum, insbesondere entlang des Kanals, dem Schaft zuführbar sein. Grundsätzlich kann der Stößel zumindest im Wesentlichen eine Länge aufweisen, die der Länge des Kanals entspricht. In einigen Ausführungsformen, insbesondere in Ausführungsformen mit einem gebogenen Schaft, kann die Länge des Stößels derart kleiner sein als die des Kanals, dass in einem vollständig eingeschobenen Zustand des Stößels ein distales Ende des Stößels zumindest im Wesentlichen bis in einen Nahbereich zu einem Beginn eines gebogenen Schaftabschnitts erstreckt. Hierbei kann der Schaft an seinem distalen Ende den gebogenen Schaftabschnitt aufweisen. Der vorgeschobene Zustand kann durch einen Anschlag eines Teils des Stößels an dem proximalen Ende des Applikators definiert sein.

Das Betätigungselement kann insbesondere dazu vorgesehen sein, dass der Aufnahmeraum verkleinerbar ist. Unter geführt bewegbar kann insbesondere verstanden werden, dass der Applikator eine Führungsvorrichtung umfasst. Insbesondere kann das Betätigungselement zumindest im Wesentlichen über einen gesamten Bewegungsumfang geführt sein, um den es in einem montierten Zustand des Applikators bewegbar ist. In dem montierten Zustand ist das Betätigungselement an dem Grundkörper vorgesehen. Beispielsweise kann das Betätigungselement auf den Grundkörper aufgesteckt werden. Das Betätigungselement kann insbesondere während des Verkleinerns zumindest im Wesentlichen kontinuierlich in Kontakt mit dem Grundkörper stehen. Das Betätigungselement kann das proximale Ende des Applikators definieren. Ferner kann das Betätigungselement, insbesondere in einem proximalen Abschnitt des Betätigungselements, den Kanal definieren. Der Kanal kann sich generell durch das Betätigungselement, insbesondere durch den proximalen Abschnitt des Betätigungselements, erstrecken. Das Betätigungselement kann an seinem proximalen Ende eine weitere Öffnung aufweisen, durch die der Stößel in den Applikator einschiebbar ist. Die weitere Öffnung kann das proximale Ende des Kanals definieren.

Der Aufnahmeraum kann dazu vorgesehen sein, dass das Mittel einfach zur Applizierung vorsehbar und/oder in den Applikator einbringbar ist. Insbesondere kann der Aufnahmeraum ein Volumen definieren, das größer ist als das Volumen des Mittels, das zur Applikation vorgesehen ist. Der Aufnahmeraum kann sich entlang der Haupterstreckungsrichtung des Grundkörpers, insbesondere entlang des Kanals, zumindest im Wesentlichen erstrecken. Ferner kann der Aufnahmeraum flach sein. Das kann bedeuten, dass der Aufnahmeraum zwei zumindest im Wesentlichen senkrecht zueinanderstehende Erstreckungsrichtungen umfasst, die den Aufnahmeraum zumindest im Wesentlichen maßgeblich definieren. In einer dritten senkrecht zu diesen Erstreckungsrichtungen stehende Erstreckungsrichtung kann sich der Aufnahmeraum über eine maßgeblich kürzere Länge erstrecken. "Flach" soll hierbei nicht auf eine ebene Ausgestaltung des Aufnahmeraums beschränkt sein. Der Aufnahmeraum kann insgesamt gekrümmt ausgebildet sein. Die genannten Erstreckungsrichtungen können sich daher ortsabhängig ändern. Insbesondere kann der Aufnahmeraum die Form eines Hohlzylindermantelabschnitts aufweisen, der sich lediglich über einen Teil eines Umfangs des betreffenden Hohlzylinders erstreckt. Ferner kann der Aufnahmeraum zumindest im Wesentlichen eine Höhe aufweisen, die zumindest im Wesentlichen dem Innendurchmesser des Kanals entspricht. Mit der Höhe kann bevorzugt die dritte Erstreckungsrichtung gemeint sein. Der Kanal kann sich entlang einer Seitenwand des Aufnahmeraums erstrecken und/oder von der Seitenwand des Aufnahmeraums zumindest teilweise definiert sein. Der Teil des Kanals, der sich durch den Aufnahmeraum erstrecken kann, kann zudem zu dem übrigen Aufnahmeraum geöffnet sein. Dadurch kann das Mittel, das in den Aufnahmeraum eingebracht ist, dem Kanal zugeführt werden. Insbesondere ist das Mittel durch das Verkleinern des Aufnahmeraums dem Kanal zuführbar. Unter Verkleinern des Aufnahmeraums kann verstanden werden, dass Wandungen des Aufnahmeraums aufeinander zu bewegbar sind. Insbesondere kann der Aufnahmeraum derart verkleinerbar sein, dass der Applikator, insbesondere der Grundkörper und/oder das Betätigungselement, nicht elastisch und/oder plastisch verformt werden muss.

Das Verkleinern wird durch die Relativbewegung zwischen dem Grundkörper und dem Betätigungselement erreicht. Die Relativbewegung kann eine geführte Bewegung und/oder eine Bewegung entlang eines Führungselements einer Führungsvorrichtung umfassen. In einem verkleinerten Zustand des Aufnahmeraums kann die Applikationsposition definiert sein. Ferner kann durch die Relativbewegung das Betätigungselement derart relativ zu dem Grundkörper bewegbar sein, dass sich der Kanal derart durch den Applikator erstreckt, dass der Stößel durch das Betätigungselement, den Grundkörper, den Aufnahmeraum und den Schaft schiebbar ist. In einer von der Applikationsposition verschiedenen Position kann sich ein Kanalabschnitt, der durch das Betätigungselement ausgebildet ist, versetzt von dem Aufnahmeraum, dem Schaft und/oder einem distalen Teil des Kanals befinden. Insbesondere kann der Stößel in der verschiedenen Position nicht derart schiebbar sein, dass das Mittel durch den Schaft bewegbar ist und/oder das Mittel applizierbar ist. Die Applikationsposition kann durch zumindest einen mechanischen Anschlag definiert sein. Beispielsweise kann das Betätigungselement bis zu der Applikationsposition relativ zu dem Grundkörper geführt bewegbar sein.

Ferner kann ein Teil des Mittels, der sich in dem Aufnahmeraum befindet, sich zeitgleich in dem Kanal befinden. Damit, dass das Mittel dem Kanal zuführbar ist, kann gemeint sein, dass zumindest im Wesentlichen ein maßgeblicher Teil des Mittels dem Kanal zuführbar ist. Beispielsweise kann in einem nicht verkleinerten Zustand der Teil des Mittels, der sich zeitgleich in dem Aufnahmeraum und dem Kanal befindet, nicht für eine vorgesehene Handlung ausreichen. Durch das Zuführen kann das mit dem Stößel beförderbare Volumen des Mittels vergrößerbar sein. Außerdem kann das Zuführen ein Komprimieren des Mittels umfassen. Beispielsweise kann durch das Verkleinern des Aufnahmeraums und/oder das Zuführen Luft aus dem Aufnahmeraum entfernbar sein. Das kann zur Folge haben, dass das Mittel kontinuierlicher mit dem Stößel beförderbar ist. Beispielsweise kann das Mittel homogener in dem Kanal angeordnet sein als bei herkömmlichen Applikatoren. Das kann bedeuten, dass zumindest im Wesentlichen geringere Lufteinschlüsse in dem und/oder zwischen dem Mittel vorkommen, insbesondere wenn der Applikator in der Applikationsposition ist. Der Benutzer spürt bei dem Bewegen des Stößels eine kontinuierliche Kraft und kann das Mittel besser dosieren.

Ferner kann der Aufnahmeraum durch die Relativbewegung zwischen dem Betätigungselement und dem Grundkörper auf einen Querschnitt verkleinerbar sein, der zumindest im Wesentlichen dem Querschnitt des Kanals entspricht. Es versteht sich, dass Fertigungstoleranzen und/oder Spiel zwischen dem Betätigungselement und dem Grundkörper vorliegen können. Insbesondere kann der Querschnitt des Kanals kreisförmig ausgebildet sein. Der Querschnitt kann zumindest im Wesentlichen einem Querschnitt des Stößels entsprechen. Vorteilhafterweise kann ausgehend von einem Zustand, in dem der Aufnahmeraum mit dem Mittel beladen ist, zumindest ein Großteil des Mittels dem Kanal zuführbar sein. Es kann eine hohe Effizienz in Bezug auf den Materialeinsatz erreichbar sein. Ferner kann insbesondere im Bereich des Aufnahmeraums der Kanal zumindest im Wesentlichen durchgängig durch Wandungen definiert sein.

Außerdem kann das Betätigungselement durch eine Relativbewegung zu dem Grundkörper in eine Beladeposition bringbar sein, wobei der Aufnahmeraum in der Beladeposition zugänglich ist. Insbesondere ist das Betätigungselement geführt in die Beladeposition bringbar. Das kann bedeuten, dass Betätigungselement wahlweise in die Betätigungselement und/oder die Applikationsposition bringbar ist. Das Betätigungselement ist beispielsweise reversibel in die Betätigungselement und/oder die Applikationsposition bringbar. Zugänglich kann bedeuten, dass der Aufnahmeraum eine Öffnung zu einer Umgebung des Applikationsinstruments aufweist. Beispielsweise kann der Benutzer das Applikationsinstrument in der Beladeposition beladen, beziehungsweise Mittel in den Aufnahmeraum einbringen. Insbesondere ist in der Beladeposition Mittel in den Aufnahmeraum einbringbar. Es können zudem weitere Positionen, insbesondere Relativpositionen zwischen dem Betätigungselement und dem Grundkörper, vorgesehen sein, in denen der Aufnahmeraum zugänglich ist. Die Beladeposition kann die Position sein, in der das Beladen zumindest im Wesentlichen am effizientesten vornehmbar ist. Die Beladeposition kann durch zumindest einen mechanischen Anschlag definiert sein. Beispielsweise können zwei Positionen vorgesehen sein, die durch mechanische Anschläge definiert sind. Die Positionen können die Applikationsposition und die Beladeposition umfassen. Das Applikationsinstrument kann durch das Vorsehen der Beladeposition besonders benutzerfreundlich und/oder (zeit-)effizient bedienbar sein.

Ferner kann das Betätigungselement in eine Einbringposition in den Grundkörper einbringbar sein, wobei die Einbringposition zumindest im Wesentlichen der Beladeposition entspricht. Die Einbringposition kann weiterhin eine Startposition für die Relativbewegung umfassen. Das Betätigungselement kann beispielsweise separat von dem Grundkörper bereitstellbar sein. Beide Teile können etwa separat in einer sterilen Umverpackung lieferbar sein. In einem Schritt in einer zeitlichen Abfolge kurz vor dem Einbringen des Mittels in den Aufnahmeraum kann es vorgesehen sein, dass das Betätigungselement in den Grundkörper eingebracht wird. Der Grundkörper kann beispielsweise an seinem proximalen Ende eine Einbringöffnung umfassen, die derart ausgebildet ist, dass das Betätigungselement zumindest abschnittsweise in den Grundkörper einbringbar ist. Vorteilhafterweise kann die Benutzerfreundlichkeit verbessert und die Bedienbarkeit des Applikators vereinfacht werden.

Für das Einbringen und/oder die Relativbewegung kann insbesondere die Führungsvorrichtung von großer Bedeutung sein. Die Führungsvorrichtung kann beispielsweise von dem Betätigungselement und dem Grundkörper gemeinsam ausgebildet sein. In einigen Ausführungsformen kann die Führungsvorrichtung ähnlich einem Bajonettverschluss funktionieren. Insbesondere kann die Führungsvorrichtung einen Vorsprung umfassen und/oder eine Führungsnut umfassen. Bei dem Durchführen der Relativbewegung kann der Vorsprung in der Führungsnut geführt sein. Die Führungsvorrichtung kann alternativ oder zusätzlich einen Abschnitt umfassen, der für das Einbringen des Betätigungselements in den Grundkörper ausgebildet ist. In der Einbringposition, der Beladeposition und/oder der Applikationsposition kann das Betätigungselement zumindest teilweise von dem Grundkörper aufgenommen sein.

Außerdem kann der Grundkörper ein Beladefenster ausbilden, durch das der Aufnahmeraum für ein Beladen mit dem Mittel zumindest in einer Beladeposition des Betätigungselements zugänglich ist. Das Beladefenster kann insbesondere Abmaße aufweisen, die ein Einbringen des Mittels in den Aufnahmeraum mittels eines medizinischen Instruments ermöglicht. Beispielsweise kann das Mittel mittels einer Pinzette, einer Spritze, eines Schlauchs, einer Gieß- und/oder Schüttvorrichtung und/oder dergleichen einbringbar sein. Ferner kann das Fenster groß genug sein, dass der Behandler mit seinen Fingern in den Aufnahmeraum hineingreifen kann. Dadurch kann ein praktischer, kompakter und/oder benutzerfreundlicher Applikator bereitgestellt werden.

Ferner kann der Grundkörper zumindest abschnittsweise hohlzylinderförmig ausgebildet sein und das Beladefenster durch einen Durchbruch durch eine Wandung des Grundkörpers ausgebildet sein. Durch das Beladefenster kann ferner ein Teil des Betätigungselements einsehbar sein. Das kann bedeuten, dass der Grundkörper einen Hohlraum aufweist, wobei in den Hohlraum des Grundkörpers das Betätigungselement einbringbar ist. Dadurch kann eine Konstruktion und/oder eine kostengünstige Herstellung des Applikators erreicht werden.

Zudem kann sich der Aufnahmeraum zumindest in einer Beladeposition unterhalb der Wandung erstrecken, sodass das Beladefenster einen ersten Abschnitt des Aufnahmeraums abdeckt und die Wandung einen zweiten Abschnitt des Aufnahmeraums abdeckt. Eine Grundfläche des Aufnahmeraums kann größer sein als eine durch das Beladefenster definierte flächige Ausprägung an der ersten Wandung. Vorteilhafterweise kann dadurch das Material effizienter und/oder effektiver nach dem Beladen in dem Aufnahmeraum gehalten werden. Die Gefahr, dass das Mittel wieder aus dem Aufnahmeraum ausläuft, kann reduziert werden.

Ferner kann die Relativbewegung ein Drehen des Grundkörpers und des Betätigungselements relativ zueinander umfassen. Das Drehen kann relativ zu einer Längsachse bzw. Mittelachse des Grundkörpers und/oder des Betätigungselements erfolgen. In anderen Worten kann das Drehen ein Drehen des Betätigungselement um eine Längsachse bzw. Mittelachse des Grundkörpers umfassen oder umgekehrt. Der Grundkörper kann das Betätigungselement bezüglich dessen Längsachse bzw. Mittelachse umfänglich umgreifen. Weiterhin kann die Relativbewegung eine zumindest im Wesentlichen ausschließliche Drehung in einer Ebene umfassen, wobei zumindest im Wesentlichen keine maßgebliche translative Relativbewegung zwischen Betätigungselement und Grundkörper vorgesehen ist. Durch das Drehen ist eine einfache Bedienbarkeit gewährleistet. Weiterhin kann dadurch eine kompakte Konstruktion des Applikators erreicht werden. In anderen Ausführungsformen kann die Relativbewegung eine translative Bewegung umfassen. Beispielsweise kann das Betätigungselement in einer Spirale relativ zu dem Grundkörper führbar sein. Mit translativ kann eine Richtung gemeint sein, die zumindest im Wesentlichen parallel zu der Haupterstreckungsrichtung verläuft.

Zudem kann der Grundkörper und das Betätigungselement korrespondierende Anschlagsflächen ausbilden, die eine maximale Vergrößerung des Aufnahmeraums definieren. Die maximale Vergrößerung des Aufnahmeraums kann insbesondere in der Beladeposition und/oder der Einbringposition vorliegen. Die Anschlagflächen können beispielsweise durch die Führungsvorrichtung ausgebildet sein. In anderen Ausführungsformen können sich die Anschlagsflächen beispielsweise entlang des Hohlraums des Grundkörpers erstrecken und/oder sich abschnittsweise entlang einer Außenfläche des Betätigungselements erstrecken. Eine Bediensicherheit kann dadurch verbessert werden. Dadurch, dass definierte Positionen vorgegeben sind, die durch einfache konstruktive Maßnahmen hergestellt werden, kann der Applikator intuitiv bedienbar sein.

Außerdem kann das Betätigungselement einen Schiebeabschnitt umfassen, der eine erste Seitenwand des Aufnahmeraums definiert, und der dazu eingerichtet ist, das Mittel bei der Relativbewegung auf den Kanal zuzuschieben und dadurch zu komprimieren, sodass es durch den Stößel beförderbar ist. Es kann durch wenige Teile ein effizienter Applikator bereitgestellt werden. Während des Zuschiebens kann zudem Luft aus dem Aufnahmeraum entfernbar sein. Das kann bedeuten, dass eine Mitteldichte in dem Aufnahmeraum und/oder dem Kanal erhöhbar ist. Der Schiebeabschnitt kann sich insbesondere von einem unteren Teil des Aufnahmeraums bis zu der Wandung des Grundkörpers erstrecken. Das Betätigungselement kann derart in dem Grundkörper einbringbar sein, dass es zumindest im Wesentlichen passgenau in dem Grundkörper vorsehbar ist. Das kann bedeuten, dass der Schiebeabschnitt die Wandung des Grundkörper kontaktieren kann. Weiterhin kann der Schiebeabschnitt die Wandung des Grundkörpers zumindest im Wesentlichen kontinuierlich während des Zuschiebens kontaktieren. Vorteilhafterweise kann dadurch erreicht werden, dass das Mittel effizient und/oder effektiv dem Kanal zuführbar ist. Der Applikator wird zumindest im Wesentlichen wenig verschmutzt.

Ferner kann der Grundkörper einen Wandabschnitt ausbilden, der eine zweite Seitenwand des Aufnahmeraums definiert. Die Seitenwand kann dem Schiebeabschnitt gegenüberliegen. Zwischen der Seitenwand und dem Schiebeabschnitt können sich seitlich weitere Seitenwände erstrecken, die zumindest im Wesentlichen nicht aufeinander zu bewegbar sind. Durch das Verkleinern des Aufnahmeraums können die weiteren Seitenwände verkleinerbar sein. In vorteilhafter Weise kann ein kostengünstiger Applikator bereitgestellt werden. Dieser kommt mit wenigen Teilen aus und weist eine einfache Konstruktion auf.

Ferner können die erste Seitenwand und die zweite Seitenwand in der Applikationsposition gemeinsam einen Abschnitt des Kanals ausbilden. Die erste Seitenwand und/oder die zweite Seitenwand kann jeweils konkav ausgebildet sein. Insbesondere kann die erste Seitenwand und/oder die zweite Seitenwand eine zumindest im Wesentlichen halbkreisförmige Krümmung und/oder einen zumindest im Wesentlichen halbkreisförmigen Querschnitt aufweisen. Die erste Seitenwand und die zweite Seitenwand können in der Applikationsposition gemeinsam einen zylinderförmigen Kanal definieren. Ferner können die erste Seitenwand und die zweite Seitenwand in der Applikationsposition gemeinsam eine kreisförmige Öffnung zu dem zylinderförmigen Kanal definieren. Der zylinderförmige Kanal kann abschnittsweise den Kanal definieren. Die Seitenwände, die in zumindest einer Relativposition den relativ großen Aufnahmeraum definieren können, können vorteilhafterweise in einer anderen Relativposition den relativ kleinen Kanal ausbilden. Dadurch kann ein besonders kompakter Applikator realisierbar. Dieser kann weiterhin effizient zu bedienen sein.

Außerdem kann das Betätigungselement zumindest in einer Beladeposition einen Boden des Aufnahmeraums definieren. Das Mittel kann derart in den Aufnahmeraum einbringbar sein, dass es den Boden zumindest im Wesentlichen bedeckt. Beispielsweise ist der Applikator dazu vorgesehen, während des Beladens waagerecht gehalten zu werden. In einer waagerechten Position, insbesondere wenn der Aufnahmeraum von oben zugänglich ist, stellt der Boden die untere Fläche des Aufnahmeraums dar. Der Boden kann insbesondere in der Beladeposition eine größere Fläche aufweisen als die Seitenflächen. Ferner kann der Boden eine größere flächige Ausdehnung aufweisen als das Beladefenster. Der Boden ist vorzugsweise insgesamt gekrümmt. Der Boden kann maßgeblich durch das Verkleinern des Aufnahmeraums verkleinerbar sein. Der Boden kann abschnittsweise durch die Außenfläche des Betätigungselements definiert sein. Insbesondere weist das Betätigungselement eine gekrümmte Außenfläche auf, die den Boden ausbildet. Diese gekrümmte Außenfläche kann im Wesentlichen einem Abschnitt eines Zylindermantels entsprechen. Die Außenfläche kann relativ zu der zweiten Seitenfläche des Aufnahmeraums bewegbar sein. Beispielsweise kann die zweite Seitenfläche an die Außenfläche angrenzen. Die Außenfläche kann unter zumindest im Wesentlichen kontinuierlichem Kontakt an der zweiten Seitenfläche vorbeibewegt werden. Insbesondere kann der den Boden definierende Teil der Außenfläche an der zweiten Seitenfläche vorbeibewegt werden. Es kann dadurch ein effizienter und/oder kompakter Applikator bereitgestellt werden.

Zudem kann das Betätigungselement einen Halteabschnitt umfasst, der aus dem Grundkörper herausragt, und der dazu eingerichtet ist, von einem Benutzer gehalten zu werden, während er die Relativbewegung durchführt. Dadurch können etwa die zueinander beweglich geführten Teile des Applikators gegriffen und/oder mechanisch fixiert werden. Dadurch kann der Applikator mechanisch manipulierbar sein. Der Halteabschnitt kann beispielsweise eine profilierte, insbesondere geriffelte, Oberfläche aufweisen. Der Halteabschnitt kann auch als Betätigungsabschnitt bezeichnet werden. Dadurch kann eine Griffigkeit des Betätigungselements verbessert werden. In einigen Ausführungsformen weist das Betätigungselement einen distalen Abschnitt mit einem ersten Durchmesser auf, der in den Hohlraum des Grundkörpers einführbar ist, sowie einen proximalen Abschnitt mit einem zweiten Durchmesser, der größer ist als der erste Durchmesser und/oder als ein Innendurchmesser des Hohlraums. Der zweite Abschnitt kann den Halteabschnitt umfassen und/oder ausbilden. Das Betätigungselement kann insgesamt pilzförmig ausgebildet sein. In einem in den Hohlraum eingeschobenen Zustand kann eine Verbreiterung des Betätigungselements, insbesondere des Halteabschnitts, an einem proximalen Ende des Grundkörpers anliegen, das eine Zugangsöffnung zu dem Hohlraum ausbildet. Mittelachsen des Grundkörpers und des Betätigungselements können im eingesetzten Zustand koaxial angeordnet sein.

Außerdem kann der Grundkörper einstückig ausgebildet sein und/oder das Betätigungselement einstückig ausgebildet sein. Beispielsweise kann der Grundkörper und/oder das Betätigungselement mittels eines Gießverfahrens, eines Fräsverfahrens und/oder mittels eines additiven Fertigungsverfahrens herstellbar sein. Insbesondere der Grundkörper und/oder das Betätigungselement ein Kunststoffteil und/oder insbesondere aus PVC (Polyvinylchlorid), PET (Polyethylenterephthalat) und PP (Polypropylen) ausgebildet sein. Der Applikator kann kostengünstig und/oder effizient herstellbar sein.

Zudem kann der Stößel ein Führungselement umfassen, das dazu eingerichtet ist, eine Rotation des Stößels während des Applizierens zu verhindern und den Stößel entlang des Kanals zu führen. Der Stößel kann einen Förderstab umfassen, der dazu eingerichtet ist, in den Kanal eingeschoben zu werden. Das kann bedeuten, dass der Stößel teilweise in den Kanal einschiebbar ist. Das Führungselement kann sich zumindest im Wesentlichen parallel zu dem Förderstab erstrecken. Insbesondere ist das Führungselement stabförmig und/oder rohrförmig. Ferner kann der Förderstab an einem proximalen Ende des Stößels mit dem Führungselement beispielsweise über ein Basiselement, insbesondere eine Verbindungsplatte, verbunden sein. In einem vollständig eingeschobenen Zustand des Stößels, insbesondere des Förderstabs, kann das Basiselement an dem Betätigungselement anliegen. Dadurch kann der Förderstab stets zuverlässig wieder aus dem Kanal herausgezogen werden.

Zudem kann das Betätigungselement und/oder der Grundkörper einen Führungskanal ausbilden und das Führungselement in den Führungskanal einbringbar sein. Ferner kann das Führungselement in dem Führungskanal führbar sein. Insbesondere kann der Führungskanal länglich ausgebildet sein. Zudem kann sich der Führungskanal abschnittsweise durch das Betätigungselement und den Grundkörper erstrecken. Außerdem kann sich der Führungskanal durch ein Drehzentrum der relativen Drehbewegung zwischen dem Betätigungselement und dem Grundkörper erstrecken Der Führungskanal ist insbesondere koaxial mit der Längsachse bzw. Mittelachse des Grundkörpers und/oder des Betätigungselements angeordnet. Insbesondere kann sich der Führungskanal entlang einer Drehachse und/oder eines Drehzentrums des Betätigungselements und des Grundkörpers erstrecken. Es kann dadurch ein besonders bediensicherer und kompakter Applikator bereitgestellt werden. Die Gefahr vor einer Fehlbedienung und/oder einer Fehlfunktion kann reduziert werden.

Zudem kann das Betätigungselement in der Applikationsposition verrastbar sein. Insbesondere kann der Applikator eine Arretiervorrichtung umfassen, die dazu eingerichtet ist, das Betätigungselement relativ zu dem Grundkörper in der Applikationsposition zu halten. Die Arretiervorrichtung kann zumindest teilweise durch den Grundkörper und/oder das Betätigungselement ausgebildet sein. Ferner kann die Arretiervorrichtung ein Rastelement und einen Rastabschnitt umfassen, wobei das Rastelement dazu eingerichtet ist, in dem Rastabschnitt einzurasten. Insbesondere kann das Rastelement an dem Grundkörper angeordnet sein und/oder gemeinsam mit dem Grundkörper bewegbar sein. Der Rastabschnitt kann an dem Betätigungselement angeordnet sein. Das Rastelement kann beispielsweise eine federgespannte Rastkugel umfassen und/oder der Rastabschnitt eine zu der Rastkugel zumindest im Wesentlichen passende kugelförmige Ausnehmung umfassen. Das Rastelement kann gegen eine Außenfläche des Betätigungselements vorgespannt sein. In der Applikationsposition kann das Betätigungselement derart verrastet sein, dass für eine Bewegung des Betätigungselements aus der Applikationsposition eine derart hohe Kraft aufgebracht werden muss, dass das Rastelement, insbesondere radial, aus dem Rastabschnitt bewegbar ist. Dadurch kann eine Vorspannkraft des Rastelements erhöhbar sein.

Es ist zudem ein Verfahren zum Beladen eines erfindungsgemäßen medizinischen Applikators bereitgestellt. Das Verfahren umfasst die Schritte Einbringen eines Mittels in den Aufnahmeraum und Bewegen des Grundkörpers und des Betätigungselements relativ zueinander, um den Aufnahmeraum zu verkleinern.

Ferner kann das Verfahren den Schritt Einbringen des Betätigungselements in den Grundkörper umfassen.

Im Folgenden wird die vorliegende Erfindung anhand der beigefügten Figuren beispielhaft beschrieben. Die Zeichnung, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es zeigen:
- Fig. 1: eine schematische Seitenansicht eines medizinischen Applikators ohne Stößel;
- Fig. 2: eine schematische Seitenansicht des Stößels;
- Fig. 3: eine schematische Seitenansicht eines Grundkörpers;
- Fig. 4: eine schematische Ansicht des Grundkörpers von der proximalen Seite;
- Fig. 5: eine schematische perspektivische Ansicht des Grundkörper von der proximalen Seite;
- Fig. 6: eine schematische Seitenansicht eines Betätigungselements;
- Fig. 7: eine schematische Ansicht des Betätigungselements von der distalen Richtung aus;
- Fig. 8: eine schematische Ansicht des Betätigungselements von der proximalen Richtung aus;
- Fig. 9: eine schematische Seitenansicht des Applikators in einer Applikationsposition;
- Fig. 10: eine schematische Schnittansicht des Applikators in der Applikationsposition von der proximalen Seite;
- Fig. 11: eine schematische Seitenansicht des Applikators in einer Beladeposition;
- Fig. 12: eine schematische Schnittansicht des Applikators in der Beladeposition von der proximalen Seite; und
- Fig. 13: ein schematisches Ablaufdiagramm eines Verfahrens für das Beladen des Applikators.

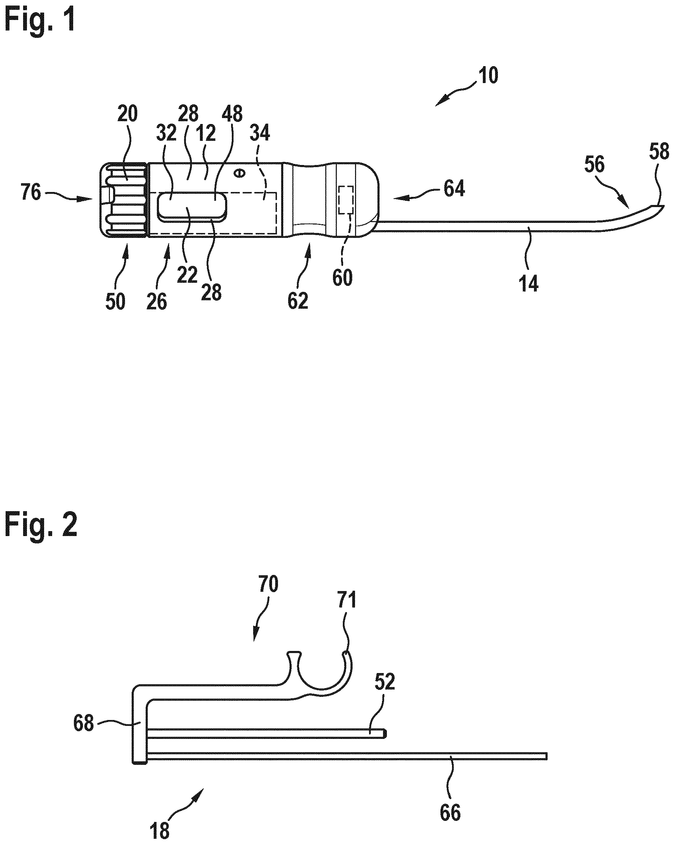

In der Fig. 1 ist eine schematische Seitenansicht eines medizinischen Applikators 10 ohne Stößel 18 gezeigt. Der zu dem medizinischen Applikator zugehörige Stößel 18 ist in Fig. 2 in einer schematischen Seitenansicht gezeigt. Bezugnehmen auf beide Figuren wird die Funktionsweise des medizinischen Applikators 10 im Folgenden beschrieben.

Der medizinische Applikator 10 ist zum Applizieren eines Mittels vorgesehen. Das Mittel ist in dem dargestellten Fall ein knorpelhaltiges Stoffgemisch, das insbesondere zur Durchführung des "Minced Cartilage"-Verfahrens vorgesehen ist. Der Applikator 10 umfasst einen Grundkörper 12, einen Schaft 14 und ein Betätigungselement 20 und ist in einem Zustand dargestellt, in dem das Betätigungselement 20 an bzw. teilweise in dem Grundkörper 12 angeordnet ist. Der Applikator 10 ist grundsätzlich dazu eingerichtet, von einem Benutzer während des Applizierens mit einer Hand gehalten zu werden. Die Hand des Benutzers kann den Grundkörper 12 beispielsweise zumindest teilweise umgreifen bzw. umschließen. Zur ergonomischen Handhabung weist der Grundkörper 12 beispielsweise eine umfängliche Ausnehmung 62 in einem Nahbereich zu seinem distalen Ende 64 auf. Die Ausnehmung 62 ist als eine umfängliche Verjüngung ausgebildet, durch die die Handhabung des Applikators 10 sicherer gemacht ist.

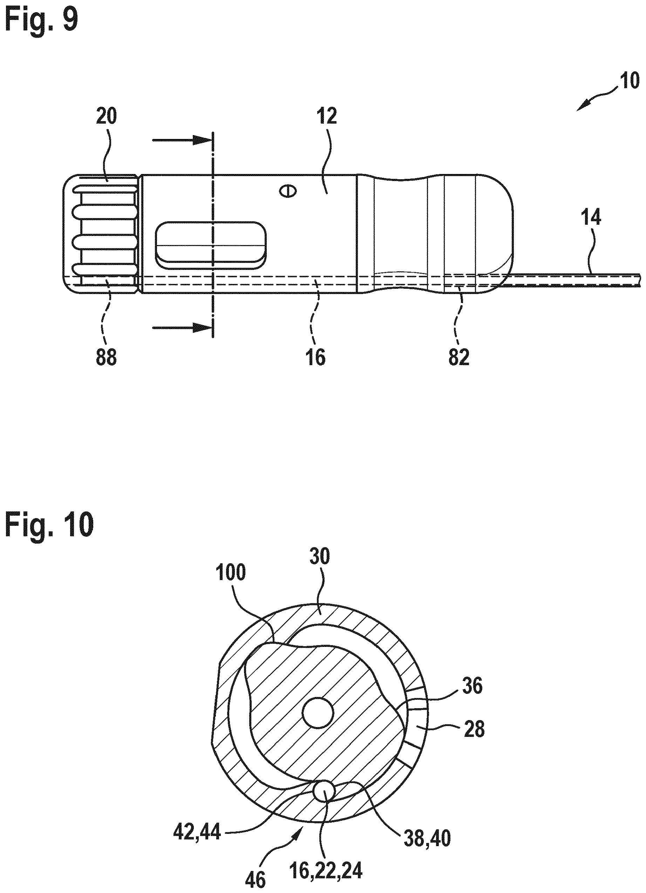

Es sei insbesondere auf die Figuren 9 bis 12 verwiesen. In diesen ist zu erkennen, dass das Betätigungselement 20 und der Grundkörper 12 verschiedene Relativpositionen zueinander einnehmen können. Die hierin beschriebenen Merkmale können insbesondere von den Relativpositionen abhängen, wie es in dem Zusammenhang den genannten Figuren beschrieben ist.

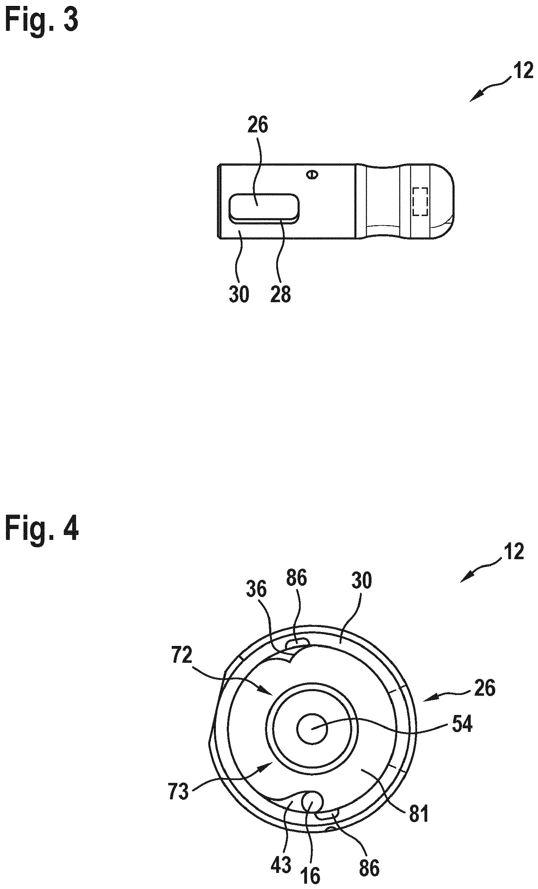

Der Grundkörper 12 und das Betätigungselement 20 sind aus einem Kunststoff gefertigt und einstückig ausgebildet. Ferner ist der Grundkörper 12 abschnittsweise hohlzylinderförmig ausgebildet. Insbesondere ist ein proximaler Abschnitt 74 des Grundkörpers 12 hohlzylinderförmig ausgebildet und definiert einen Hohlraum 72. Der Hohlraum 72 bzw. der proximale Abschnitt 74 erstreckt sich von einem proximalen Ende 76 des Grundkörpers 12 ausgehend über 70 % der Länge des Grundkörpers 12. Der Hohlraum 72 ist radial durch eine Wandung 28 begrenzt. An dem proximalen Ende 76 weist der Grundkörper 12 eine Öffnung 78 auf, durch die das Betätigungselement 20 zumindest abschnittsweise und/oder teilweise in den Grundkörper 12, insbesondere den Hohlraum 72, einbringbar ist. Der Grundkörper 12 ist in den Fig. 3 bis 5 in verschiedenen Ansichten besser gezeigt. Bezüglich einiger Merkmale sei auf diese Figuren verwiesen. Ferner bildet der Grundkörper 12 ein Beladefenster 26 aus, das durch einen Durchbruch 28 durch die Wandung 28 des Grundkörper 12 ausgebildet ist. Durch das Beladefenster 26 ist ein Aufnahmeraum 22 zugänglich. Es versteht sich, dass dies insbesondere lediglich für die in der Fig. 1 gezeigten Relativposition gilt. Der Aufnahmeraum 22 ist dazu eingerichtet, das Mittel aufzunehmen. Wie in den folgenden Figuren genauer beschrieben, ist er Aufnahmeraum 22 gemeinsam durch den Grundkörper 12 und das Betätigungselement 20 ausgebildet. Gewissermaßen entsteht der Aufnahmeraum 22, wenn das Betätigungselement 20 in den Hohlraum 72 eingebracht ist. Durch das Beladefenster 26 ist der Aufnahmeraum 22 für ein Beladen mit dem Mittel zugänglich. Dazu weist das Beladefenster 26 geeignete Abmessungen auf. Beispielsweise ist der gezeigte Grundkörper 12 in etwa so groß wie die Hand des Behandlers. Der gezeigte Grundkörper 12 ist beispielhaft 12 cm in seiner Haupterstreckungsrichtung lang. Das Beladefenster ist beispielhaft 4 cm in die Haupterstreckungsrichtung lang und 2 cm breit. Beliebige andere Abmessungen sind denkbar und können insbesondere abhängig von dem zu verwendenden Mittel sein.

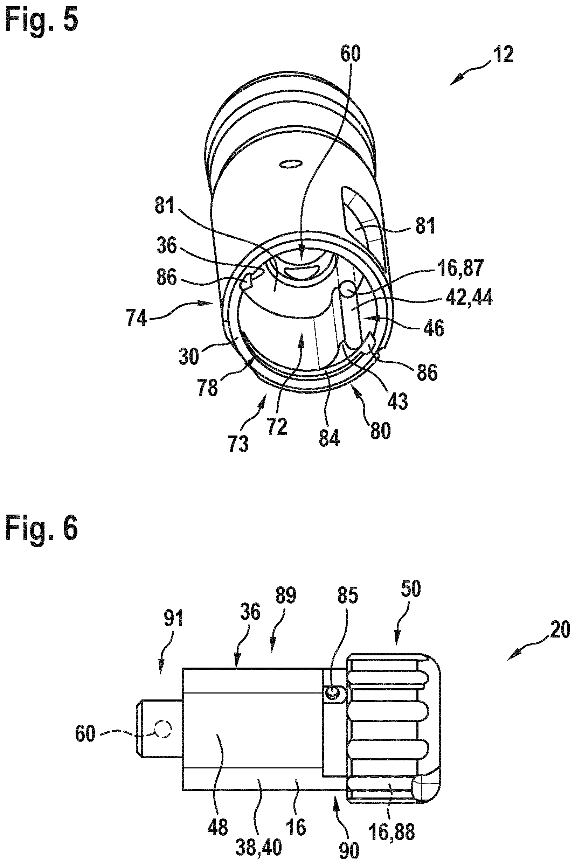

Ferner ist zu erkennen, dass sich der Aufnahmeraum 22 unterhalb der Wandung 30 erstreckt. Abschnittsweise kann die Wandung 30 den Aufnahmeraum 22 in die radiale Richtung begrenzen. Das Beladefenster 26 deckt einen ersten Abschnitt 32 des Aufnahmeraums 22 ab und die Wandung 30 einen zweiten Abschnitt 34 des Aufnahmeraum 22. An dem proximalen Ende 76 ist der Aufnahmeraum 22, wie er in der Fig. 1 zu sehen ist, durch das Betätigungselement 20 abschnittsweise begrenzt und an dem distalen Ende 64 durch den Grundkörper 12. Der Grundkörper 12 umfasst dazu einen Vorsprung 81, der in den Fig. 4 und 5 ersichtlich ist. Nach radial innen ist der Aufnahmeraum 22 durch einen Boden 48 definiert, der durch das Betätigungselement 20 definiert ist.

Das Betätigungselement 20 ragt teilweise über das proximale Ende des Grundkörper 12 hinaus. Insbesondere ragt ein Halteabschnitt 50 des Betätigungselements 20 aus dem Grundkörper in die proximale Richtung hinaus. Der Halteabschnitt 50 ist dazu eingerichtet, von dem Benutzer gehalten zu werden, wenn er die Relativbewegung durchführt. Dazu ist der Halteabschnitt 50 profiliert ausgebildet. Der Halteabschnitt 50 weist in periodischen Abständen Vertiefungen auf, die eine Griffigkeit des Halteabschnitts 50 verbessern. Außerdem weist der Halteabschnitt 50 einen größeren Durchmesser auf als der Hohlraum 72 des Grundkörpers 12. in dem eingeführten Zustand des Betätigungselements 20 in den Hohlraum 72 überragt der Halteabschnitt 50 den Grundkörper dadurch zumindest teilweise. Das heißt, der Halteabschnitt 50 definiert einen mechanischen Anschlag, der in dem eingeführten Zustand den Grundkörper 12 kontaktiert. Der Halteabschnitt 50 kann insbesondere gemeinsam mit einer Führungsvorrichtung 80 zusammenwirken, die im Zusammenhang mit den Fig. 5 und 6 beschrieben ist.

Zudem umfasst der Applikator 10 eine Arretiervorrichtung 60, die dazu eingerichtet ist, das Betätigungselement relativ zu dem Grundkörper 12 in einer Applikationsposition zu halten. Die Arretiervorrichtung 60 ist teilweise durch den Grundkörper 12 und das Betätigungselement 20 ausgebildet. Die Arretiervorrichtung 60 umfasst ein nicht näher gezeigtes Rastelement und einen nicht näher gezeigten Rastabschnitt, wobei das Rastelement dazu eingerichtet ist, in dem Rastabschnitt einzurasten. Das Rastelement ist an dem Grundkörper 12 angeordnet. In der Fig. 5 ist ein zur Montage des Rastelements vorgesehenes Montageloch zu sehen. Der Rastabschnitt ist an dem Betätigungselement 20 angeordnet sein. Das Rastelement umfasst eine federgespannte Rastkugel und der Rastabschnitt eine zu der Rastkugel zumindest im Wesentlichen passende kugelförmige Ausnehmung. Das Rastelement ist in radialer Richtung nach innen wirkend gegen eine Außenfläche des Betätigungselements 20 vorgespannt.

An dem distalen Ende 64 umfasst der Grundkörper 12 eine Schaftmontageausnehmung 82 (Fig. 9), die sich entlang der Haupterstreckungsrichtung des Grundkörpers 12 erstreckt. In der Schaftmontageausnehmung 82 ist der Schaft 14 angeordnet. Der Schaft 14 ist stabförmig und hohlzylinderförmig ausgebildet und umfasst einen Abschnitt eines Kanals, der in der Fig. 1 nicht näher gezeigt ist. Der über den Grundkörper 12 hinausragende Teil des Schafts 14 ist in etwa so lang wie der Grundkörper 12. An seinem distalen Bereich 56 weist der Schaft 14 eine Biegung auf. Zudem weist der Schaft 14 an dem distalen Ende eine Öffnung 58 auf, die ein distales Ende des Kanals darstellt. Der Kanal ist dazu eingerichtet, dass das Mittel durch den Kanal beförderbar ist und einer Applikationsstelle zuführbar ist. Beispielsweise kann die Applikationsstelle ein defekter Knorpel sein. Das distale Ende, insbesondere die Öffnung 58 kann in einen Nahbereich des Defektes gebracht werden und das Mittel dem Defekt zuführbar sein. Das kann bedeuten, dass das Mittel auf dem Defekt appliziert wird. Der Kanal erstreckt sich zumindest durch den Schaft 14 und den Grundkörper 12 hindurch, wie in den folgenden Figuren genauer zu sehen ist. Der Schaft 14 ist derart gebogen, dass das Mittel mit einem Winkel von 20° bezogen auf eine Längsachse des Schafts 14 applizierbar ist.

In der Applikationsposition erstreckt sich der Kanal durch den Schaft 14, den Grundkörper 12 und das Betätigungselement 20. Diese Bauteile weisen zumindest abschnittsweise Bohrungen, Löcher, Durchgänge und/oder dergleichen auf, die in der Applikationsposition konzentrisch zueinander angeordnet sind. Weiterhin kann in der Applikationsposition der Stößel 18 (Fig. 2) in den Kanal eingeschoben werden, um das Mittel zu applizieren. Der Stößel 18 umfasst einen Förderstab 66, der in den Kanal einschiebbar ist, ein Führungselement 52, einen Handhabungsabschnitt 70 und ein Basiselement 68. Über das Basiselement 68 sind die der Förderstab 66, der Handhabungsabschnitt 70 und das Führungselement 52 miteinander verbunden. Insbesondere kann das Basiselement 68 dem Stößel 18 notwendige mechanische Stabilität verleihen. Mittels des Handhabungsabschnitts 70 kann der Benutzer den Stößel 18 bedienen. Insbesondere wenn der Förderstab 66 in den Kanal eingeführt ist, kann der Benutzer mittels des Handhabungsabschnitts 70 das Mittel zu der Öffnung 58 befördern. Dazu weist der Handhabungsabschnitt 70 eine halboffene Schelle 71 auf, die der Benutzer zur Handhabung greifen kann. Für das Befördern drückt der Benutzer den Stößel 18 von der proximalen Seite in Richtung des distalen Endes des Applikators 10. Das Führungselement 52 ist in einen Führungskanal einbringbar und dazu eingerichtet, eine Rotation des Stößels 18 während des Applizierens zu verhindern und den Stößel 18, insbesondere den Förderstab 66, entlang des Kanals zu führen. Wäre das Führungselement 52 nicht vorgesehen, könnte beispielsweise ein Moment, das der Benutzer während des Applizierens durch das Bedienen des Handhabungsabschnitts 70 bewirkt, eine Drehung des Stößels 18 um den Förderstab 66 herum bewirken. Dies ist zumindest im Wesentlichen durch das Führungselement 52 verhindert. Der zu dem Führungselement 52 zugehörige Führungskanal ist in den folgenden Figuren näher gezeigt. Das Führungselement 52 weist einen etwas größeren Durchmesser als der Förderstab 66 auf und ist kürzer. Insbesondere ist das Führungselement 52 in etwa so lang wie das gemeinsam in dem eingeschobenen Zustand des Betätigungselements 20 durch das Betätigungselement 50 und den Grundkörper 12 ausgebildete Objekt. In einem vollständig eingeschobenen Zustand des Stößels 18 in den Kanal stößt das Basiselement 68 an das distale Ende des Betätigungselements 20 an. Derart ist ein weiteres Vorschieben des Förderstabs 66 verhindert.

In den Figuren 3, 4 und 5 sind einige Merkmale des Grundkörpers 12, die bereits im Zusammenhang mit den vorhergehenden Figuren beschrieben wurden, besser zu erkennen. Die Fig. 3 zeigt eine schematische Seitenansicht des Grundkörpers 12, die Fig. 4 zeigt eine schematische Ansicht des Grundkörpers 12 von der proximalen Seite 76 und die Fig. 5 zeigt eine schematische perspektivische Ansicht des Grundkörper 12 von der proximalen Seite 76. in den Fig. 4 und Fig. 5 kann man insbesondere in den hohlzylinderförmigen Grundkörper 12 einsehen. Insbesondere sind Merkmale zu erkennen, die den Aufnahmeraum 22 definieren. Grundsätzlich erkennt man, dass der Grundkörper 12 in seinem proximalen Ende offen ausgebildet ist und eine proximale Öffnung definiert. Durch die proximale Öffnung ist das Betätigungselement 20 einführbar. Man erkennt die Wandung 30 des Grundkörpers 12. Das Beladefenster 26 ist als Durchbruch 28 durch die Wandung 30 ausgebildet. Ferner bildet der Grundkörper 12 den Vorsprung 81 aus, der insbesondere in einer der Applikationsposition unterschiedlichen Position eine distale Seitenwand des Aufnahmeraums 22 definiert. Der Vorsprung 81 definiert eine umfängliche Fläche, die zumindest im Wesentlichen senkrecht zu der Haupterstreckungsrichtung des Grundkörpers 12 angeordnet ist. Der Kanal 16 verläuft in die Haupterstreckungsrichtung und ist zumindest im Wesentlichen senkrecht zu dem Vorsprung 81 angeordnet. Der Grundkörper 12 bildet abschnittsweise den Kanal 16 aus. In dem Vorsprung 81 ist ein Loch 87 angeordnet, das entlang des Kanals 16 verläuft und den Kanal 16 abschnittsweise ausbildet. Das Loch 87 kann gemeinsam mit der Schaftmontageausnehmung 82 ausgebildet sein und/oder sich bis zu dem Schaft 14 erstrecken, wenn der Schaft 14 in dem Grundkörper 12 angeordnet ist. Ferner ist das Loch 87 konzentrisch mit dem Schaft 14 und/oder der Schaftmontageausnehmung 82 angeordnet. Ausgehend von dem Loch 87 erstreckt sich in Richtung des proximalen Endes des Grundkörpers 12 ein Wandabschnitt 42, der eine zweite Seitenwand des Aufnahmeraums 22 definiert. Der Wandabschnitt 42 bildet in der Applikationsposition einen Abschnitt 46 des Kanals 16 aus. Der Abschnitt 46 erstreckt sich entlang des Wandabschnitts 42. Der Wandabschnitt 42 ist kanalseitig konvex und zumindest im Wesentlichen halbrund ausgebildet. Ein Radius der Krümmung, die den kanalseitigen Abschnitt definiert, entspricht zumindest im Wesentlichen einem Radius des Lochs 87 und des Kanals 16. Der Wandabschnitt 42 ist an einem Vorsprung 43 angeordnet, der von der Wandung 30 in den Hohlraum 72 hineinragt.

An dem proximalen Ende grenzt der Wandabschnitt 42 an einen Teil der Führungsvorrichtung 80 an. Insbesondere grenzt der Wandabschnitt 42 an eine Führungsnut 84 der Führungsvorrichtung 80 an. Die Führungsnut 84 erstreckt sich umfangseitig an einer Innenfläche der Wandung 30 über etwa 25 % des gesamten Umfangs. Ferner verläuft die Führungsnut 84 entlang eines gedachten Kreises, der zumindest im Wesentlichen senkrecht zu dem Kanal 16 angeordnet ist. An einem Ende der Führungsnut 84 weist die Führungsvorrichtung 80 eine Einführnut 86 auf, die sich zumindest im Wesentlichen senkrecht zu der Führungsnut 84 bis an das proximale Ende der Wandung 30 erstreckt. Durch die Einführnut 86 ist bereichsweise ein Radius der proximalen Öffnung 73 erweitert. Umfangseitig gegenüberliegend sind zwei der Einführnuten 86 und Führungsnuten 84 angeordnet, die gleich ausgebildet sind.

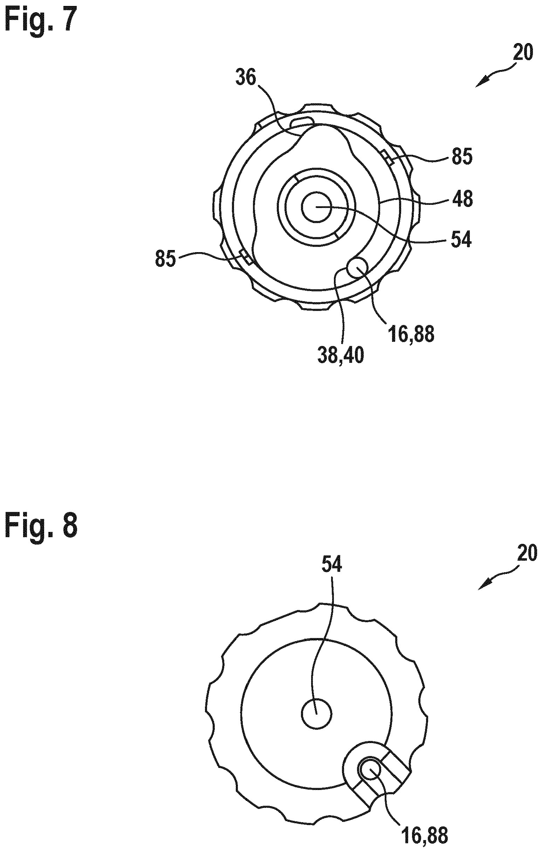

Die Führungsvorrichtung 80, insbesondere die Einführnut 86 ist dazu eingerichtet, dass das Betätigungselement 20 in eine Einbringposition, die eine relative Position des Betätigungselements 20 zu dem Grundkörper 12 definiert, in den Grundkörper 12 einbringbar ist. In der Einbringposition weist der Aufnahmeraum 22 eine maximale Vergrößerung auf. Zugleich definiert die Einbringposition eine Beladeposition, in der der Aufnahmeraum 22 durch das Beladefenster 26 für ein Beladen mit dem Mittel zugänglich ist. Durch das zweite Ende der Führungsnut 86 ist die Applikationsposition definiert. Die Führungsnut 86 ist dazu eingerichtet, das Betätigungselement 20 während einer Bewegung zu führen. Die Bewegung ist insbesondere ein Drehen des Betätigungselements 20. Das Betätigungselement kann von einer Beladeposition, insbesondere der Einbringposition, zu der Applikationsposition gedreht werden. Die Führungsvorrichtung 80 ist zudem teilweise von dem Betätigungselement 20 ausgebildet. An dem Betätigungselement 20 sind, wie in den Fig. 6 und 7 ersichtlich, Führungselemente 85, insbesondere Führungsnasen und/oder Führungsnutfedern, ausgebildet. Die Führungselemente 85 definieren gemeinsam mit den Einführnuten 86 die relative Drehposition zwischen Grundkörper 12 und Betätigungselement 20, in der das Betätigungselement 20 in den Hohlraum 72 einbringbar ist. Ferner definieren die Führungselemente 85 und die Führungsnuten 86 eine axiale Vorschubposition des Betätigungselements 20 in dem Hohlraum 72. Außerdem führen die Führungsnuten 86 gemeinsam mit den Führungselementen 85 das Betätigungselement 20.

Die Position mit der maximalen Vergrößerung des Aufnahmeraums 22, insbesondere die Einbringposition, ist zudem durch korrespondierende Anschlagsflächen 36 definiert. Eine der Anschlagsflächen 36 ist an einem Vorsprung angeordnet, der von dem Grundkörper 12 ausgebildet ist und in den Hohlraum 72 hineinragt.

Ferner ist in dem Zentrum des Grundkörpers 12 ein Teil des Führungskanals 54 angeordnet (siehe dazu Fig. 4).

In den Fig. 6, 7 und 8 ist das Betätigungselement 20 in verschiedenen Ansichten gezeigt. Die Fig. 6 zeigt eine schematische Seitenansicht des Betätigungselements 20, die Fig. 7 zeigt eine schematische Ansicht des Betätigungselements 20 von der distalen Richtung aus und die Fig. 8 zeigt eine schematische Ansicht des Betätigungselements 20 von der proximalen Richtung aus.

Das Betätigungselement 20 umfasst den Handhabeabschnitt 50, den Basisabschnitt 90, den Raumabschnitt 89 und den Arretierabschnitt 60. in einigen Ausführungsformen kann das Betätigungselement 20 insgesamt pilzförmig sein. Der Handhabeabschnitt 50 ist bereits in dem Zusammenhang mit der Fig. 1 beschrieben. Zusätzlich ist in dem Handhabeabschnitt ein Loch 88 ausgebildet, das in der Applikationsposition konzentrisch mit dem Kanal 16 angeordnet ist. Durch das Loch 88 ist der Förderstab 66 in den Kanal 16 einschiebbar. Ferner weist das Loch 88 zumindest im Wesentlichen denselben Durchmesser wie das Loch 87 auf.

Der Basisabschnitt 90 schließt distal an den Handhabeabschnitt 50 an. In dem Zustand, in dem das Betätigungselement 20 in den Hohlraum 72 eingeführt ist, schließt der Basisabschnitt 90 zumindest im Wesentlichen bündig mit dem proximalen Ende des Grundkörpers 12. Der Basisabschnitt 90 definiert das proximale Ende des Aufnahmeraums 22 und umfasst die Führungselemente 85. Die Führungselemente 85 ragen umfangseitig von dem Basisabschnitt 90 weg. Der Basisabschnitt ist zumindest im Wesentlichen kreisförmig ausgebildet und ist passgenau zu dem Hohlraum 72 ausgebildet. Dadurch kann eine Dichtigkeit des Aufnahmeraums 22 gewährleistet sein. Der Basisabschnitt 90 ist zudem durch eine Fortführung des Lochs 88 durchbrochen. Das Loch 88 erstreckt sich durch den Basisabschnitt 90 und den Handhabeabschnitt 50. Eine distale Fläche des Basisabschnitts 90 schließt in eingeführten Zustand zumindest im Wesentlichen bündig mit dem proximalen Ende des Vorsprungs 43. Das Loch 88 wird in der Applikationsposition durch den Wandabschnitt 42 und einen Schiebeabschnitt 38 fortgeführt. Der Schiebeabschnitt 38 ist von dem Betätigungselement 20 längs des Raumabschnitts 89 ausgebildet. Ferner definiert der Schiebeabschnitt 38 eine erste Seitenwand des Aufnahmeraums 22. Der Schiebeabschnitt 38 ist ähnlich dem Wandabschnitt 42 ausgebildet. Gemeinsam bilden der Schiebeabschnitt 38 und der Wandabschnitt 42 in der Applikationsposition den Kanal 16 abschnittsweise aus und haben definieren gemeinsam ein Loch mit zumindest im Wesentlichen dem gleichen Durchmesser wie das Loch 87 und das Loch 88. Der Schiebeabschnitt 38 ist insbesondere dazu eingerichtet, das Mittel bei der Relativbewegung auf den Kanal 16 zuzuschieben. Der Kanal 16 erstreckt sich entlang des Wandabschnitts 42 und wird teilweise durch den Wandabschnitt 42 ausgebildet. Durch das Zuschieben wird das Mittel derart komprimiert, dass es durch den Stößel 18, insbesondere den Förderstab 66, beförderbar ist.

Ferner bildet der Raumabschnitt 89 eine der korrespondierenden Wandungen 36 aus, die mit der entsprechenden Wandung 36 des Grundkörpers 12 wechselwirken kann.

Distal schließt sich der Arretierabschnitt 91 an. Das Betätigungselement 20 bildet in dem Arretierabschnitt 91 teilweise die Arretiervorrichtung 60 aus. Insbesondere bildet das Betätigungselement 20 einen Rastabschnitt, bevorzugt eine kugelförmige Ausnehmung, aus. Von dem Grundkörper 12 kann ein weiterer Teil der Arretiervorrichtung 60 ausgebildet und/oder aufgenommen sein. Der Grundkörper weist, siehe Fig. 5, eine Bohrung auf, in der beispielsweise eine federgespannte Kugel angeordnet ist. Die Kugel wird durch eine Vorspannung radial nach innen gegen den Arretierabschnitt 91 des Betätigungselements 20 gedrückt. In der Applikationsposition wird die Kugel in die kugelförmige Ausnehmung des Betätigungselements 20 gedrückt. In der Applikationsposition kann das Betätigungselement 20 dadurch verrastbar sein.

Der Raumabschnitt 89 ist abschnittsweise, wie etwa in der Fig. 7 ersichtlich, verjüngt und weist einen kleineren Radius als der Basisabschnitt 90 auf. Dadurch wird der Boden 48 definiert und eine Aussparung für den Aufnahmeraum 22 bereitgestellt. Der Aufnahmeraum 22 ist in einer Beladeposition nach radial außen durch das abschnittsweise durch das Beladefenster 28 und die Wandung 30 und nach radial innen durch das Betätigungselement 20, insbesondere den Boden 48, teilweise definiert.

Ferner bildet das Betätigungselement 20 in seinem Zentrum (Fig. 7 und Fig. 8) abschnittsweise den Führungskanal 54 aus. Durch das proximale Ende des Betätigungselement 20 ist das Führungselement 52 in den Führungskanal 54 einbringbar. Der betätigungselementseitige Abschnitt des Führungskanals 54 ist konzentrisch mit dem grundkörperseitigen Abschnitt des Führungskanals 54 angeordnet. Weiterhin weisen beide Abschnitte zumindest im Wesentlichen den gleichen Durchmesser auf.

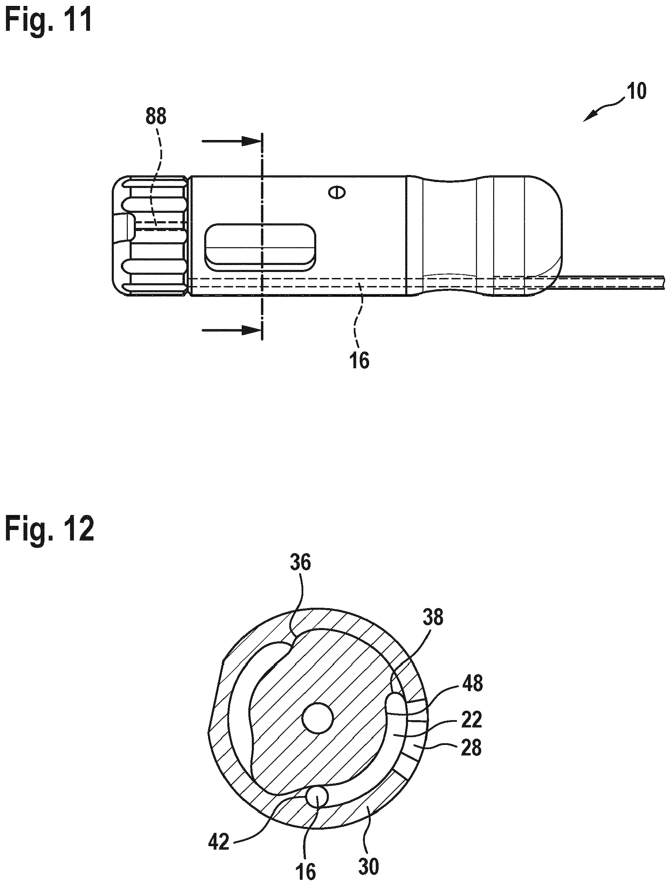

In den Fig. 9 bis 12 ist der Applikator 10 in der Applikationsposition (Fig. 9 und 10) und in einer Beladeposition (Fig. 11 und 12), insbesondere der Position mit maximaler Vergrößerung des Aufnahmeraums 22, gezeigt. Die Fig. 9 zeigt eine schematische Seitenansicht des Applikators 10 in der Applikationsposition, die Fig. 10 zeigt eine schematische Schnittansicht des Applikators 10 in der Applikationsposition von der proximalen Seite, die Fig. 11 zeigt eine schematische Seitenansicht des Applikators 10 in einer Beladeposition und die Fig. 12 zeigt eine schematische Schnittansicht des Applikators 10 in der Beladeposition von der proximalen Seite. Aus den Fig. 9 und 11 ist jeweils ersichtlich, welche Schnittebene in den zugehörigen Schnittansichten gezeigt wird.

In der Applikationsposition erstreckt sich der Kanal 16 durch den Schaft 14, durch den Grundkörper 12 und das Betätigungselement 20. Das Loch 88 des Betätigungselements 20 ist konzentrisch mit dem Kanal 16 angeordnet. Das Betätigungselement 20, insbesondere das Loch 88, kann durch ein Drehen des Betätigungselements 20 aus dieser Position bewegt werden und insbesondere in eine Beladeposition gebracht werden. In der Beladeposition (siehe Fig. 11 und 12) ist der Aufnahmeraum 22 durch das Beladefenster 28 zugänglich und mit dem Mittel beladbar, das Loch 88 ist aber nicht konzentrisch zu dem Kanal angeordnet. In dieser Position kann der Stößel 18 nicht in den Kanal 16 eingeführt werden und das Mittel nicht mittels des Stößels 18 befördert werden. In der Applikationsposition (Fig. 10) ist der Aufnahmeraum 22 verkleinert und nicht durch das Beladefenster 28 zugänglich.

Man sieht in der Fig. 10, dass die erste Seitenwand 40, insbesondere der Schiebeabschnitt 38, und die zweite Seitenwand 44, insbesondere der Wandabschnitt 42, in der Applikationsposition gemeinsam einen Abschnitt 46 des Kanals 16 ausbilden. Aus einer der Beladepositionen ist der Aufnahmeraum 22 derart verkleinert worden, indem der Benutzer das Betätigungselement 20 geführt gedreht hat, dass sein Querschnitt 24 zumindest im Wesentlichen dem Querschnitt des Kanals 16 entspricht. Ferner ist durch das Drehen das Mittel, das von dem Aufnahmeraum 22 aufgenommen ist, komprimiert worden und/oder dem Kanal 16 zugeführt worden. Dadurch ist das Mittel, insbesondere effizient, durch den Stößel 18 beförderbar. Ferner stoßen zwei korrespondierende Wandungen 100 des Grundkörpers 100 und des Betätigungselements 20 aneinander. Zudem ist das Betätigungselement 20 in der Applikationsposition verrastet.

In der Fig. 12 ist der Applikator 10 außerdem in der Einbringposition gezeigt. In der gezeigten Position stehen die korrespondierenden Anschlagsflächen 36 in Kontakt, wodurch eine maximale Vergrößerung des Aufnahmeraums definiert ist. In dieser Position kann der Benutzer Mittel in den Aufnahmeraum 22 einbringen. Danach kann der Benutzer das Mittel durch das Drehen dem Kanal 16 zuführen. Man erkennt, dass der Aufnahmeraum 22 durch den Schiebeabschnitt 38, den Wandabschnitt 42, den Boden 48, die Wandung 30 und das Beladefenster 28 definiert ist. Durch das Drehen wird der Abschnitt im Bodenbereich und Wand- und Beladefensterbereich verringert, der den Aufnahmeraum definiert. In der Applikationsposition ist der Aufnahmeraum zumindest im Wesentlichen lediglich durch den Wandabschnitt 42 und den Schiebeabschnitt 38 definiert. Der Aufnahmeraum 22 ist also durch das Drehen in der Größe, insbesondere in dem Volumen, veränderbar. Ferner erkennt man, dass das Betätigungselement 20 zumindest im Wesentlichen derart passgenau in den Hohlraum 72 des Grundkörpers eingebracht, dass eine für den Betrieb notwendige Dichtigkeit erreicht ist.

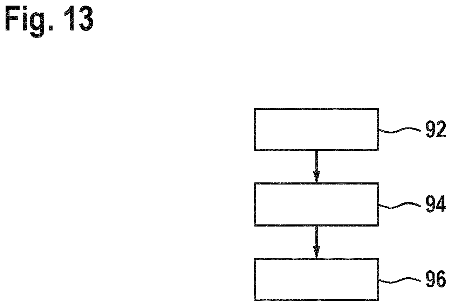

Die Fig. 13 zeigt ein schematisches Ablaufdiagramm eines Verfahrens für das Beladen des Applikators 10. Bezüglich einer detaillierten Beschreibung sei auf die Beschreibung im Zusammenhang mit den vorhergehenden Figuren verwiesen. Das im Folgenden beschriebene Verfahren kann in einer beliebigen, sinnvollen Reihenfolge ausgeführt werden und/oder einzelne Schritte ausgelassen werden. Das Verfahren umfasst einen Schritt 92 eines Einbringens eines Mittels in den Aufnahmeraum 22, einen Schritt 94 eines Bewegens des Grundkörpers 12 und des Betätigungselements 20 relativ zueinander, um den Aufnahmeraum 22 zu verkleinern, und einen Schritt 96 eines Einbringens des Betätigungselements 20 in den Grundkörper 12.

### Bezugszeichenliste

- 10: Medizinischer Applikator;
- 12: Grundkörper;
- 14: Schaft;
- 16: Kanal;
- 18: Stößel;
- 20: Betätigungselement;
- 22: Aufnahmeraum;
- 24: Querschnitt;
- 26: Beladefenster;
- 28: Durchbruch;
- 30: Wandung;
- 32: erster Abschnitt;
- 34: zweiter Abschnitt;
- 36: Anschlagsflächen;
- 38: Schiebeabschnitt;
- 40: erste Seitenwand;
- 42: Wandabschnitt;
- 43: Vorsprung;
- 44: zweite Seitenwand;
- 46: Abschnitt;
- 48: Boden;
- 50: Halteabschnitt;
- 52: Führungselement;
- 54: Führungskanal;
- 56: distaler Endbereich;
- 58: Öffnung;
- 60: Arretiervorrichtung;
- 62: Ausnehmung;
- 64: distales Ende;
- 66: Förderstab;
- 68: Basiselement;
- 70: Handhabungsabschnitt;
- 72: Hohlraum;
- 73: proximale Öffnung;
- 74: proximaler Abschnitt;
- 76: proximales Ende;
- 78: Öffnung;
- 80: Führungsvorrichtung;
- 81: Vorsprung;
- 82: Schaftmontageausnehmung;
- 84: Führungsnut;
- 85: Führungselement;
- 86: Einführnut;
- 87: Loch;
- 88: Loch;
- 89: Raumabschnitt;
- 90: Basisabschnitt;
- 91: Arretierabschnitt;
- 92: Schritt;
- 94: Schritt;
- 96: Schritt; und
- 100: korrespondierende Wandungen.

## Patentansprüche

1. Medizinischer Applikator (10) zum Applizieren eines Mittels, umfassend:
einen Grundkörper (12);
einen an dem Grundkörper (12) angeordneten Schaft (14), wobei der Grundkörper (12) und der Schaft (14) einen Kanal (16) ausbilden, der sich durch den Grundkörper (12) und den Schaft (14) hindurch erstreckt und durch den das Mittel einer Applikationsstelle zuführbar ist;
einen Stößel (18), der in den Kanal (16) einschiebbar ist, um das Mittel zu applizieren,
ein Betätigungselement (20), das an dem Grundkörper (12) geführt bewegbar ist; und
einen Aufnahmeraum (22), der von dem Grundkörper (12) und/oder dem Betätigungselement (20) ausgebildet ist und der dazu eingerichtet ist, das Mittel aufzunehmen;
wobei das Betätigungselement (20) durch eine Relativbewegung zu dem Grundkörper (12) in eine Applikationsposition bringbar ist, wobei der Aufnahmeraum (22) durch die Relativbewegung zwischen dem Betätigungselement (20) und dem Grundkörper (12) verkleinerbar ist, und wobei in den Aufnahmeraum (22) eingebrachtes Mittel durch das Verkleinern des Aufnahmeraums (22) dem Kanal (16) zuführbar ist.

2. Medizinischer Applikator (10) nach Anspruch 1,
wobei der Aufnahmeraum (22) durch die Relativbewegung zwischen dem Betätigungselement (20) und dem Grundkörper (12) auf einen Querschnitt (24) verkleinerbar ist, der zumindest im Wesentlichen dem Querschnitt des Kanals (16) entspricht.

3. Medizinischer Applikator (10) nach Anspruch 1 oder 2,
wobei das Betätigungselement (20) durch eine Relativbewegung zu dem Grundkörper (12) in eine Beladeposition bringbar ist, wobei der Aufnahmeraum (22) in der Beladeposition zugänglich ist.

4. Medizinischer Applikator nach Anspruch 3,
wobei das Betätigungselement (20) in eine Einbringposition in den Grundkörper (12) einbringbar ist, wobei die Einbringposition zumindest im Wesentlichen der Beladeposition entspricht.

5. Medizinischer Applikator (10) nach einem der vorhergehenden Ansprüche,
wobei der Grundkörper (12) ein Beladefenster (26) ausbildet, durch das der Aufnahmeraum (22) für ein Beladen mit dem Mittel zumindest in einer Beladeposition des Betätigungselements (20) zugänglich ist.

6. Medizinischer Applikator (10) nach Anspruch 5,
wobei der Grundkörper (12) zumindest abschnittsweise hohlzylinderförmig ausgebildet ist, und wobei das Beladefenster (26) durch einen Durchbruch (28) durch eine Wandung (30) des Grundkörpers (12) ausgebildet ist.

7. Medizinischer Applikator (10) zumindest nach Anspruch 5 und 6,
wobei sich der Aufnahmeraum (22) zumindest in einer Beladeposition unterhalb der Wandung (30) erstreckt, sodass das Beladefenster (26) einen ersten Abschnitt (32) des Aufnahmeraums (22) abdeckt und die Wandung (30) einen zweiten Abschnitt (34) des Aufnahmeraums (22) abdeckt.

8. Medizinischer Applikator (10) nach einem der vorhergehenden Ansprüche,
wobei die Relativbewegung ein Drehen des Grundkörpers (12) und des Betätigungselements (20) relativ zueinander umfasst.

9. Medizinischer Applikator (10) nach einem der vorhergehenden Ansprüche,
wobei der Grundkörper (12) und das Betätigungselement (20) korrespondierende Anschlagsflächen (36) ausbilden, die eine maximale Vergrößerung des Aufnahmeraums (22) definieren.

10. Medizinischer Applikator (10) nach einem der vorhergehenden Ansprüche,
wobei das Betätigungselement (20) einen Schiebeabschnitt (38) umfasst, der eine erste Seitenwand (40) des Aufnahmeraums (22) definiert, und der dazu eingerichtet ist, das Mittel bei der Relativbewegung auf den Kanal (16) zuzuschieben und dadurch zu komprimieren, sodass es durch den Stößel (18) beförderbar ist.

11. Medizinischer Applikator (10) nach einem der vorhergehenden Ansprüche,
wobei der Grundkörper (12) einen Wandabschnitt (42) ausbildet, der eine zweite Seitenwand (44) des Aufnahmeraums (22) definiert.

12. Medizinischer Applikator (10) zumindest nach Anspruch 10 und 11,
wobei die erste Seitenwand (40) und die zweite Seitenwand (44) in der Applikationsposition gemeinsam einen Abschnitt (46) des Kanals (16) ausbilden.

13. Medizinischer Applikator (10) nach einem der vorhergehenden Ansprüche,
wobei das Betätigungselement (20) zumindest in einer Beladeposition einen Boden (48) des Aufnahmeraums (22) definiert.

14. Medizinischer Applikator (10) nach einem der vorhergehenden Ansprüche,
wobei das Betätigungselement (20) einen Halteabschnitt (50) umfasst, der aus dem Grundkörper (12) herausragt, und der dazu eingerichtet ist, von einem Benutzer gehalten zu werden, während er die Relativbewegung durchführt.

15. Medizinischer Applikator (10) nach einem der vorhergehenden Ansprüche,
wobei der Grundkörper (12) einstückig ausgebildet ist und/oder wobei das Betätigungselement (20) einstückig ausgebildet ist.

16. Medizinischer Applikator (10) nach einem der vorhergehenden Ansprüche,
wobei der Stößel (18) ein Führungselement (52) umfasst, das dazu eingerichtet ist, eine Rotation des Stößels (18) während des Applizierens zu verhindern und den Stößel (18) entlang des Kanals (16) zu führen.

17. Medizinischer Applikator (10) nach Anspruch 16,
wobei das Betätigungselement (20) und/oder der Grundkörper (12) einen Führungskanal (54) ausbildet, und
wobei das Führungselement (52) in den Führungskanal (54) einbringbar ist.

18. Medizinischer Applikator (10) nach einem der vorhergehenden Ansprüche, wobei das Betätigungselement (20) in der Applikationsposition verrastbar ist.

19. Verfahren zum Beladen eines medizinischen Applikators (10) nach einem der vorhergehenden Ansprüche, umfassend die Schritte:
Einbringen eines Mittels in den Aufnahmeraum (22); und
Bewegen des Grundkörpers (12) und des Betätigungselements (20) relativ zueinander, um den Aufnahmeraum (22) zu verkleinern.

20. Verfahren zum Beladen eines medizinischen Applikators (10) nach Anspruch 19, ferner umfassend den Schritt:
Einbringen des Betätigungselements (20) in den Grundkörper (12).
